# EUROPÄISCHE PATENTANMELDUNG

(11) **EP 3 895 652 A1**
(43) Veröffentlichungstag der Anmeldung: **20.10.2021**
(21) Anmeldenummer: 20170249.5
(22) Anmeldetag: 17.04.2020
(51) Int. Cl.: A61B 90/00, A61B 90/40, A61G 15/00

(54) **SCHUTZVORRICHTUNG ZUM SCHUTZ VOR AEROSOLAUSBREITUNG**

(71) Anmelder: Sandmair, Klaus, 86316 Friedberg (DE); Koch, Raimar, 83435 Bad Reichenhall (DE)
(72) Erfinder: Sandmair, Klaus, 86316 Friedberg (DE); Koch, Raimar, 83435 Bad Reichenhall (DE)
(74) Vertreter: SSM Sandmair

(57) **Zusammenfassung**

Die Erfindung betrifft eine Schutzvorrichtung zum Schutz vor Aerosolausbreitung bei medizinischen Behandlungen am Kopf, insbesondere bei zahnmedizinischen Behandlungen. Die Schutzvorrichtung umfasst einen Überkopfschutz zur Verhinderung einer Ausbreitung von Aerosolen nach oben, einen nach unten offenen Seitenschutz, der einen Behandlungsraum an einem Seitenumfang begrenzt, um eine seitliche Ausbreitung der Aerosole zu verhindern, und ein Gestell zum Positionieren des Überkopfschutzes und des Seitenschutzes relativ zu einer Patientenauflage eines Behandlungsstuhls in einer Behandlungsposition und Halten des Überkopfschutzes und des Seitenschutzes in der Behandlungsposition.

## Beschreibung

Die Erfindung betrifft eine Schutzvorrichtung zum Schutz vor Aerosolausbreitung bei medizinischen Behandlungen am Kopf, insbesondere bei zahnmedizinischen Behandlungen. Die Schutzvorrichtung umfasst einen Überkopfschutz, einen nach unten offenen Seitenschutz und ein Gestell zum Positionieren des Überkopfschutzes und des Seitenschutzes relativ zu einer Patientenauflage in einer Behandlungsposition und zum Halten des Überkopfschutzes und des Seitenschutzes in der Behandlungsposition. Der Seitenschutz begrenzt einen Behandlungsraum seitlich und ist dazu ausgebildet, die seitliche Ausbreitung von Aerosolen zu verhindern. Der Überkopfschutz ist dazu ausgebildet die Ausbreitung von Aerosolen aus dem Behandlungsraum nach oben zu verhindern.

Bei zahnmedizinischen Behandlungen werden durch einen Zahnarzt oder dessen Personal diagnostische, therapeutische und/oder präventive Maßnahmen im Mundraum von Patienten vorgenommen. Hierfür muss der Patient den Mund für einen längeren Zeitraum geöffnet halten, wobei es über die Dauer der zahnmedizinischen Behandlung regelmäßig zu einem erhöhten Speichelfluss beim Patienten kommt. Für gewöhnlich wird der Speichel durch eine oder mehrere Absaugvorrichtungen, welche direkt im Mund des Patienten angeordnet werden, abgeführt. Durch die Benutzung von Behandlungsgeräten, wie etwa Zahnbohrern und Zahnschleifern, entstehen Aerosole, welche sich in Form eines Sprühnebels ausbreiten. Unter Aerosolen versteht man ein heterogenes Gemisch aus festen und/oder flüssigen Schwebeteilchen in einem Gas. Diese im Mundraum des Patienten entstehenden Aerosole können Träger von Krankheitserregern, wie beispielsweise Viren, Keimen und/oder Bakterien sein. Die Aerosole können nicht vollständig durch die lokale Absaugvorrichtung unmittelbar im Mund abgesaugt werden und breiten sich um Mundraum des Patienten aus.

Der Erfindung liegt die Aufgabe zu Grunde, eine Vorrichtung bereitzustellen, welche die Ausbreitung von Aerosolen während einer medizinischen Behandlung am Kopf eines Patienten verhindert oder zumindest reduziert.

Die Aufgabe wird mit den Merkmalen des Anspruchs 1 gelöst. Vorteilhafte Weiterbildungen ergeben sich aus den abhängigen Ansprüchen, der Beschreibung und den Figuren.

Eine erfindungsgemäße Schutzvorrichtung umfasst einen Überkopfschutz, einen nach unten offenen Seitenschutz und ein Gestell. Der Überkopfschutz und der Seitenschutz verhindern eine nach oben und seitlich gerichtete Ausbreitung von Aerosolen aus einem Behandlungsraum, welcher an einem Seitenumfang durch den Seitenschutz begrenzt wird. Das Gestell ist dazu ausgebildet, den Überkopfschutz und den Seitenschutz relativ zu einer Patientenauflage eines Behandlungsstuhls, beispielsweise einer Kopfstütze, in einer Behandlungsposition zu positionieren und den Überkopfschutz und den Seitenschutz in der Behandlungsposition zu halten, vorteilhafterweise zu fixieren.

Der Behandlungsraum ist nach unten offen. Der Seitenschutz weist also eine untere Öffnung auf. Die untere Öffnung ist der Patientenauflage, wie etwa einer Kopfstütze des Behandlungsstuhls, auf welcher der Kopf des Patienten bei der Behandlung ruht, zugewandt. Der Behandlungsraum ist ausreichend groß, so dass zumindest der Kopf des Patienten in der Behandlungsposition von unten in den Behandlungsraum ragen kann. Bevorzugt ist der Seitenschutz über seine gesamte untere Stirnseite offen.

Der Überkopfschutz, welcher die Ausbreitung von Aerosolen nach oben verhindert oder zumindest reduziert, liegt der unteren Öffnung des Seitenschutzes während einer Behandlung in einem vertikalen Abstand gegenüber. Vorzugsweise ist der Überkopfschutz an oder sehr nahe der oberen Stirnseite des Seitenschutzes angeordnet. Der Überkopfschutz kann den Behandlungsraum nach oben zumindest teilweise begrenzen, insbesondere zumindest teilweise abdecken. Bevorzugt schließt der Überkopfschutz den Behandlungsraum nach oben vollständig. Der Überkopfschutz kann einen oberen Bereich des Behandlungsraums auch seitlich begrenzen, also eine Seitenbegrenzung zusätzlich zum Seitenschutz bilden.

Der Behandlungsraum kann insbesondere zylinderförmig, beispielsweise kreiszylinderförmig oder ellipsenzylinderförmig, sein. In anderen Ausführungen kann der Seitenschutz und/oder der Überkopfschutz aber auch so geformt sein, dass der Behandlungsraum glockenförmig ist oder sich nach oben verengt, beispielsweise konisch verengt.

Der Seitenschutz ist in zweckmäßigen Ausführungen von außen nach innen in den Behandlungsraum durchgreifbar. Der Arzt und/oder ärztliches Personal kann in derartigen Ausführungen mit den Händen von außen, von der Seite her, durch den Seitenschutz in den Behandlungsraum greifen, um den Patienten im Kopfbereich im Behandlungsraum behandeln zu können.

Der Überkopfschutz kann in einem oder mehreren Gelenken relativ zum Gestell verstellbar sein. Bei dem jeweiligen Gelenk kann es sich beispielsweise um ein Drehgelenk, wie etwa ein Scharnier, ein Kugelgelenk oder ein Schubgelenk, wie etwa ein Teleskopgelenk, handeln. Es ist auch denkbar, dass das jeweilige Gelenk mehrere Freiheitsgrade der Beweglichkeit hat, beispielsweise ein Dreh-Schub-Gelenk ist. Vorteilhaft ist, wenn der Überkopfschutz relativ zum Gestell rotatorisch und translatorisch verstellt werden kann.

Der Überkopfschutz und/oder der Seitenschutz kann oder können jeweils direkt oder indirekt mit dem Gestell lösbar verbunden sein. Sie können jeweils mit dem Gestell einzeln lösbar verbunden sein. In einer bevorzugten Alternative sind der Seitenschutz fest oder lösbar am Überkopfschutz angebracht und der Überkopfschutz lösbar mit dem Gestell verbunden. Ebenso können der Überkopfschutz fest oder lösbar am Seitenschutz angebracht und der Seitenschutz lösbar mit dem Gestell verbunden sein. Überkopfschutz und Seitenschutz können auch Teilbereiche einer integralen Schutzvorrichtung und als Einheit lösbar mit dem Gestell verbunden sein. Die jeweilige Verbindung ist in vorteilhaften Ausführungen ohne Werkzeug lösbar.

Ungeachtet einer optionalen Lösbarkeit vom Gestell kann/können der Überkopfschutz und/oder der Seitenschutz mehrteilig sein, wobei die einzelnen Elemente eines mehrteiligen Überkopfschutzes und/oder eines mehrteiligen Seitenschutzes bevorzugt lösbar miteinander verbunden sind. Anders formuliert kann/können der Überkopfschutz und/oder Seitenschutz zerstörungsfrei zerlegbar sein. In vorteilhaften Ausführungen ist/sind der Überkopfschutz und/oder Seitenschutz werkzeuglos zerlegbar. Eine einfache und rasche Zerlegbarkeit ist für den Alltagsbetrieb von Vorteil, um die Schutzvorrichtung zwischen aufeinander folgenden Behandlungen einfach und rasch sterilisieren zu können. Eine Zerlegbarkeit ist darüber für eine gründliche Reinigung und Sterilisierung vorteilhaft.

Der Überkopfschutz kann ringförmig oder teilringförmig als Ringstruktur ausgebildet sein. Der Überkopfschutz kann stattdessen beispielsweise auch eine plane oder dreidimensional gewölbte, schalenförmige Struktur sein.

In Weiterbildungen weist der Überkopfschutz eine Absaugeinrichtung auf, um Luft, insbesondere die aerosolhaltige Luft, aus dem Behandlungsraum abzusaugen.

Die Absaugeinrichtung kann ringförmig oder teilringförmig, beispielsweise als Rohrstruktur, ausgebildet sein. Von der Form unabhängig kann die Absaugeinrichtung mehrteilig ausgebildet sein. Die einzelnen Teilabschnitte der Absaugeinrichtung, beispielsweise Rohrabschnitte, können an ihrer Trenn- bzw. Verbindungsstelle lösbar, insbesondere werkzeuglos lösbar, miteinander verbunden sein. Beispielsweise kann die jeweilige Trenn- bzw. Verbindungsstelle als Steck-, Klick- und/oder Bajonettverbindung ausgebildet sein. Jedenfalls kann die Absaugeinrichtung insgesamt oder teilweise desinfizierbar und/oder austauschbar sein.

Die Absaugeinrichtung umfasst eine oder mehrere Absaugöffnungen, durch welche Luft, insbesondere die aerosolhaltige Luft aus dem Behandlungsraum, in die Absaugeinrichtung einströmen kann. Ist die Absaugöffnung schlitzförmig, kann bei entsprechend großer Bogenlänge des Schlitzes eine einzige Absaugöffnung ausreichen. Bevorzugt weist die Absaugeinrichtung jedoch eine Mehrzahl von Absaugöffnungen auf, die beispielsweise als einfache Löcher oder anders geformte Perforation einer Fluidleitung gebildet sein können. Die eine oder die mehreren Absaugöffnungen kann oder können lediglich bereichsweise an der Absaugeinrichtung vorgesehen sein. Ist die Absaugeinrichtung mehrteilig, kann ein oder können mehrere Teilabschnitte jeweils eine oder mehrere Absaugöffnungen aufweisen und ein oder mehrere andere Teilabschnitte frei von Absaugöffnungen sein.

Der Überkopfschutz dient in bevorzugten Ausführungen als Träger für den Seitenschutz. Der Seitenschutz kann insbesondere hängend am Überkopfschutz angeordnet sein.

Bevorzugt erstreckt sich die Absaugeinrichtung zumindest teilweise entlang eines oberen Rands des Seitenschutzes. Dabei kann die Absaugeinrichtung den Träger für den Seitenschutz bilden. Der Seitenschutz kann am Überkopfschutz, insbesondere an der Absaugeinrichtung, unlösbar oder, bevorzugter, lösbar befestigt sein.

Die Absaugeinrichtung kann mehrere Schlauchelemente und/oder Rohrelemente aufweisen. Beispielsweise kann die Absaugeinrichtung in einem Anschlussbereich, der vorteilhafterweise aus dem Behandlungsraum herausragt, über eine Schlauch- oder Rohrverbindung mit einer Pumpe, welche die Luft in der Absaugeinrichtung ansaugt, verbunden oder verbindbar sein. Die Absaugeinrichtung kann die Pumpe und/oder einen Filter umfassen. Der Filter ist bevorzugt im Luftstrom angeordnete, um Viren, Keime, Bakterien oder andere Krankheitserreger aus der aerosolhaltigen Luft zu filtern. Vorteilhafterweise ist der Filter austauschbar und/oder desinfizierbar. Alternativ oder zusätzlich kann die Absaugeinrichtung an eine externe Pumpe und/oder einen externen Filter angeschlossen werden. Beispielsweise kann die Absaugeinrichtung dazu eingerichtet sein, dass sie an eine Pumpe und/oder einen Filter eines Behandlungsstuhls angeschlossen werden kann und auf vorhandene Systeme hinsichtlich der Saugleistung angepasst ist. Die Kompatibilität erleichtert die Integration der Schutzvorrichtung gleich bei der Herstellung von Behandlungsstühlen und auch die Nachrüstung von bereits im Gebrauch befindlichen Behandlungsstühlen. Schlauch- und/oder Rohrelemente der Absaugeinrichtung können, außerhalb des Behandlungsraums, im Gestell verlegt sein, beispielsweise ein integraler Bestandteil des Gestells sein. Alternativ oder zusätzlich können Schlauch- und/oder Rohrelemente der Absaugeinrichtung außen am Gestell befestigt sein und/oder frei durch den Raum verlegt werden.

Der Überkopfschutz kann eine Abdeckung aufweisen, welche den Behandlungsraum an seiner oberen Stirnseite zumindest teilweise abdeckt. Mit anderen Worten kann der Seitenschutz durch die Abdeckung des Überkopfschutzes oben zumindest teilweise abgedeckt werden. Die Abdeckung ist vorzugsweise über die gesamte obere Öffnung des Seitenschutzes transparent und/oder weist einen oder mehrere transparente Teilbereiche auf, so dass der Arzt während der Behandlung durch die Abdeckung hindurch in den Behandlungsraum schauen kann. Vorzugsweise besteht die Abdeckung zumindest teilweise aus Glas und/oder Plexiglas, oder weist zumindest Bestandteile aus Glas und/oder Plexiglas auf.

Die Abdeckung kann einteilig oder mehrteilig ausgebildet sein. Bei einer mehrteiligen Abdeckung sind die einzelnen Elemente der Abdeckung bevorzugt lösbar miteinander verbunden, so dass die Abdeckung zerlegt werden kann, bevorzugt ohne Werkzeug. Die Abdeckung kann eine flache, insbesondere plattenförmige, und/oder gewölbte, insbesondere haubenartige, Form haben. Auch hybride Formen der Abdeckung sind denkbar. Beispielsweise kann der Randbereich der Abdeckung eine flache, vorzugsweise plattenförmig, und der zentrale Bereich eine gewölbte Form umfassen. Bei Ausführungsformen, bei welchen die Abdeckung eine gewölbte Form aufweist, ist die Wölbung vorzugsweise nach außen bzw. oben gerichtet. Dadurch weist der Behandlungsraum, welcher nach oben durch eine gewölbte Abdeckung begrenzt wird, ein größeres Volumen auf als ein Behandlungsraum, welcher nach oben durch eine flache, beispielsweise plane, Abdeckung begrenzt wird.

Die Abdeckung kann ein optisches Vergrößerungselement, beispielsweise eine Lupe, aufweisen, um den zu behandelnden Körperbereichs des Patienten zur Unterstützung der Behandlung optisch zu vergrößern. Das optische Vergrößerungselement kann ein integraler Bestandteil der Abdeckung sein. Beispielsweise kann eine Lupe, analog dem Prinzip einer Gleitsichtbrille, in die Abdeckung eingelassen sein. Alternativ kann das optische Vergrößerungselement und/oder ein optional weiteres optisches Vergrößerungselement beweglich an der Abdeckung oder der Absaugeinrichtung oder unabhängig von zu sterilisierenden Teilen des Überkopfschutzes am Gestell angeordnet sein. In derartigen Ausführungen kann das (weitere) optische Vergrößerungselement zwischen einer ersten Position und einer zweiten Position hin und her bewegt werden. Denkbar wäre beispielsweise, dass das bewegliche Vergrößerungselement in der ersten Position die Abdeckung nicht bedeckt und in der zweiten Position einen Teil der Abdeckung bedeckt.

In Ausführungen, in welchen der Überkopfschutz eine Absaugeinrichtung und eine Abdeckung umfasst, können die Absaugeinrichtung und die Abdeckung unbeweglich miteinander verbunden sein. Vorteilhafter ist es jedoch, wenn die Abdeckung relativ zur Absaugeinrichtung beweglich, insbesondere verstellbar, angeordnet ist. Alternativ oder zusätzlich kann die Abdeckung in Bezug auf den Seitenschutz beweglich, insbesondere verstellbar, angeordnet sein. Beispielsweise kann die Abdeckung über ein oder mehrere Gelenke mit der Absaugeinrichtung und/oder dem Seitenschutz oder unabhängig von der Absaugeinrichtung und dem Seitenschutz mit dem Gestell verbunden sein, so dass die Abdeckung relativ zum Seitenschutz und/oder zur Absaugeinrichtung zwischen einer ersten Position und einer zweiten Position hin und her bewegt werden kann. Denkbar ist, dass die Abdeckung in der ersten Position einen aufgeklappten Zustand einnimmt, in welchem die Abdeckung den Seitenschutz nach oben nicht abdeckt, um beispielsweise gereinigt und/oder desinfiziert werden zu können. In der zweiten Position kann die Abdeckung einen zugeklappten Zustand einnehmen, in welchem sie den Seitenschutz nach oben zumindest teilweise abdeckt.

Der Überkopfschutz kann ein oder mehrere Griffstücke umfassen. Die ein oder mehreren Griffstücke können mit der Abdeckung und/oder der Absaugeinrichtung und/oder einem oder mehreren optionalen Griffstückhaltern des Überkopfschutzes verbunden sein. In einer alternativen Ausführungsform sind die ein oder mehreren Griffstücke Bestandteile des Seitenschutzes und an diesem angeordnet.

Vorzugsweise sind die ein oder mehreren Griffstücke als Krafteinleitungselemente ausgebildet, so dass beispielsweise die Schutzvorrichtung, wenn ein Arzt an den Griffstücken zieht oder drückt, in die Behandlungsposition oder aus der Behandlungsposition heraus, bewegt werden kann. Alternativ oder zusätzlich können die ein oder mehreren Griffstücke auch dazu geeignet sein, dass die Abdeckung durch Ziehen oder durch Drücken an den Griffstücken von der ersten Position in eine zweite Position bewegt werden kann.

Bei einer weiteren Ausführungsform der Schutzvorrichtung ist der Seitenschutz sterilisierbar, insbesondere desinfizierbar, und/oder für den einmaligen Gebrauch austauschbar ausgebildet. Unabhängig davon kann der Seitenschutz elastisch verformbar und/oder zumindest bereichsweise vorhangartig ausgebildet sein. Vorteilhafterweise kann auch der gesamte Seitenschutz vorhangartig ausgebildet sein. Davon unabhängig kann der Seitenschutz beispielsweise mit einem Seitenschutzhalter des Überkopfschutzes, der Absaugeinrichtung des Überkopfschutzes und/oder mit dem Gestell der Schutzvorrichtung verbunden sein. Vorzugsweise handelt es sich bei der Verbindung um eine lösbare Verbindung, beispielsweise eine Klettverbindung und oder eine Magnetverbindung.

In Ausführungen, in welchen der Seitenschutz nur in einem oder mehreren Teilbereichen oder vollständig vorhangartig ausgebildet ist, kann der Seitenschutz oder nur der jeweilige Teilbereich Lamellen umfassen, vorzugsweise aus Lamellen bestehen, welche analog zu einem Lamellenvorhang von oben nach unten herabhängen und von außen nach innen in den Behandlungsraum durchgreifbar sind. Vorteilhafterweise sind die Lamellen desinfizierbar, insbesondere sterilisierbar, und/oder für den einmaligen Gebrauch ausgebildet.

Der Überkopfschutz und der Seitenschutz bilden in anderen Ausführungen der Schutzvorrichtung einen zusammenhängenden Bereich einer integralen Schutzhaube. Die Schutzhaube ist insbesondere nach unten offen und kann eine sich nach oben verjüngende, beispielsweise konische oder glockenförmige, Form aufweisen. Anders formuliert kann die Schutzhaube einen Durchmesser aufweisen, welcher im unteren Bereich größer ist als im oberen Bereich. Vorzugsweise weist die integrale Schutzhaube einen oder mehrere lokal geformte Durchgriffe auf, welche dazu geeignet sind, dass ein Arzt oder ärztliches Personal, während der Behandlung, von außen in den Behandlungsraum greifen kann.

Während der Behandlung können Flüssigkeiten gegen die Innenwand des Seitenschutzes spritzen und/oder an der Innenwand des Seitenschutzes kondensieren. Die Schutzvorrichtung kann daher eine Auffangrinne umfassen, welche die an der Innenwand herabfließende Flüssigkeit auffangen und/oder ableiten kann. In integraler Ausführung der Schutzvorrichtung, welche eine Schutzhaube umfasst, ist die Auffangrinne bevorzugt im unteren Bereich und an der Innenwand des Seitenschutzes angeordnet.

In Weiterbildungen umfasst die Schutzvorrichtung ein oder mehrere Leuchtelemente zur Ausleuchtung des Behandlungsraums. Das jeweilige Leuchtelement kann am Überkopfschutz, dem Gestell, und/oder dem Seitenschutz befestigt, insbesondere lösbar befestigt sein. Vorteilhafterweise ist das jeweilige Leuchtelement so ausgerichtet, dass es dazu geeignet ist, während der Behandlung, in den geöffneten Mund des Patienten zu leuchten.

Die Schutzvorrichtung kann ein oder mehrere klangerzeugende Schallelemente, beispielsweise einen oder mehrere Lautsprecher, aufweisen. Das jeweilige Schallelement kann am Überkopfschutz, dem Gestell, und/oder dem Seitenschutz befestigt, insbesondere lösbar befestigt sein. Vorzugsweise ist das jeweilige Schallelement dazu ausgebildet den Patienten beim Warten und/oder während der Behandlung mit beruhigenden Klängen zu beschallen.

Die Schutzvorrichtung kann eine oder mehrere Kameras umfassen. Die jeweilige Kamera kann insbesondere am Überkopfschutz angebracht sein, entweder in einer relativ zum Überkopfschutz fixen Ausrichtung oder vorzugsweise in Bezug auf die Ausrichtung verstellbar. Die jeweilige Kamera kann insbesondere auf den Kopf des Patienten, zweckmäßigerweise auf den zu behandelnden Kopfbereich, gerichtet oder in eine derartige Ausrichtung verstellbar angebracht sein. Die jeweilige Kamera kann beispielsweise an der Abdeckeinrichtung, falls vorhanden, oder bevorzugt an der Absaugeinrichtung, falls vorhanden, angeordnet sein.

Umfasst die Schutzvorrichtung eine oder mehrere Kameras, kann vorteilhafterweise ein Bildschirm zur Darstellung der mittels der jeweiligen Kamera erfassten Bilder vorgesehen sein. Ein derartiger Bildschirm kann Bestandteil der Schutzvorrichtung sein, was auch Ausbildungen umfasst, in denen der Bildschirm abgesetzt von der Schutzvorrichtung an geeigneter Stelle anbringbar ist. Grundsätzlich kann es sich bei dem Bildschirm aber auch um ein Standardprodukt handeln und zum Lieferumfang der Schutzvorrichtung nur eine entsprechende Software zur Kopplung der einen oder mehreren Kameras mit dem Bildschirm gehören. Mittels der einen oder mehreren Kameras und angeschlossenen Bildschirms kann die Behandlung in Echtzeit optisch erfasst und auf dem Bildschirm dargestellt werden, um den behandelnden Arzt oder dessen Personal bei der Behandlung zu unterstützen. So kann beispielsweise bei Zahnbehandlungen der Mundraum des Patienten vergrößert auf dem Bildschirm dargestellt werden. Zusätzlich oder alternativ zu einer Darstellung in Echtzeit, d. h. zeitgleich mit der Behandlung, kann die optische Erfassung mittels Kamera zur Dokumentation für den Arzt oder Patienten oder zu Lehrzwecken für Auszubildende oder Studierende oder grundsätzlich auch Kollegen dienen.

Die Schutzvorrichtung kann ein integraler oder nachträglich montierter Bestandteil eines Behandlungsstuhls, vorzugsweise eines Zahnarztstuhls, sein. So kann das Gestell der Schutzvorrichtung von vorherein am Behandlungsstuhl angeordnet oder für eine Montage der Schutzvorrichtung am Behandlungsstuhl gestaltet sein. Alternativ kann das Gestell der Schutzvorrichtung derart ausgebildet sein, dass die Schutzvorrichtung neben einem Behandlungsstuhl und unabhängig vom Behandlungsstuhl aufgestellt werden kann.

Die Erfindung betrifft nicht nur die Schutzvorrichtung als solche, sondern auch einen Behandlungsstuhl, insbesondere einem Zahnarztstuhl, mit integrierter oder nachträglich montierter Schutzvorrichtung. Gegenstand der Erfindung ist auch die Kombination eines Behandlungsstuhls, insbesondere eines Zahnarztstuhls, und einer für die Verwendung mit dem Stuhl geeigneten Schutzvorrichtung, die unabhängig vom Stuhl abgestützt ist.

Auch in den nachstehend formulierten Aspekten werden Merkmale der Erfindung beschrieben. Die Aspekte sind in der Art von Ansprüchen formuliert und können diese ersetzen. In den Aspekten offenbarte Merkmale können die Ansprüche ferner ergänzen und/oder relativieren, Alternativen zu einzelnen Merkmalen aufzeigen und/oder Anspruchsmerkmale erweitern. In Klammern gesetzte Bezugszeichen beziehen sich auf nachfolgend in Figuren illustrierte Ausführungsbeispiele der Erfindung. Sie schränken die in den Aspekten beschriebenen Merkmale nicht unter den Wortsinn als solchen ein, zeigen andererseits jedoch bevorzugte Möglichkeiten der Verwirklichung des jeweiligen Merkmals auf.
Aspekt 1 Schutzvorrichtung zum Schutz vor Aerosolausbreitung bei medizinischen Behandlungen am Kopf, insbesondere bei zahnmedizinischen Behandlungen, die Schutzvorrichtung umfassend:
   (a) einen Überkopfschutz (10) zur Verhinderung einer Ausbreitung von Aerosolen nach oben,
   (b) einen nach unten offenen Seitenschutz (21), der einen Behandlungsraum (20) an einem Seitenumfang begrenzt, um eine seitliche Ausbreitung der Aerosole zu verhindern, und
   (c) ein Gestell (6) zum Positionieren des Überkopfschutzes (10) und des Seitenschutzes (21) relativ zu einer Patientenauflage (3) eines Behandlungsstuhls (1) in einer Behandlungsposition und Halten des Überkopfschutzes (10) und des Seitenschutzes (21) in der Behandlungsposition,
   (d) wobei der Seitenschutz (21) von außen nach innen in den Behandlungsraum (20) durchgreifbar ist.
Aspekt 2 Schutzvorrichtung nach dem vorhergehenden Aspekt, wobei der Überkopfschutz (10) eine Absaugeinrichtung (12) zum Absaugen von Luft und Aerosolen aus dem Behandlungsraum (20) umfasst.
Aspekt 3 Schutzvorrichtung nach einem der vorhergehenden Aspekte, wobei der Überkopfschutz (10) eine zumindest teilweise transparente Abdeckung (11), die den Seitenschutz (21) nach oben zumindest teilweise abdeckt, umfasst.
Aspekt 4 Schutzvorrichtung nach einem der vorhergehenden Aspekte, wobei die Absaugeinrichtung (12) eine Absaugstruktur, vorzugsweise Ringstruktur, mit einer oder mehreren Absaugöffnungen (13) umfasst.
Aspekt 5 Schutzvorrichtung nach einem der vorhergehenden Aspekte in Kombination mit Aspekt 2, wobei die Absaugeinrichtung (12) an eine bezüglich der Schutzvorrichtung externe Absaugeinrichtung (4, 5), insbesondere an eine Absaugeinrichtung (4, 5) des Behandlungsstuhls (1), angeschlossen oder anschließbar ist.
Aspekt 6 Schutzvorrichtung nach einem der vorhergehenden Aspekte in Kombination mit Aspekt 2, wobei die Absaugeinrichtung (12) eine Pumpe (5) und einen Filter (4), vorzugsweise Virenfilter, umfasst.
Aspekt 7 Schutzvorrichtung nach einem der vorhergehenden Aspekte in Kombination mit Aspekt 3, wobei die Abdeckung (11) ein optisches Vergrößerungselement (14), beispielsweise eine Lupe, aufweist.
Aspekt 8 Schutzvorrichtung nach einem der vorhergehenden Aspekte, wobei der Überkopfschutz (10) aus mehreren Teilstrukturen (12.1, 12.2) zusammengesetzt ist, die lösbar, beispielsweise werkzeuglos lösbar, miteinander verbunden sind.
Aspekt 9 Schutzvorrichtung nach einem der vorhergehenden Aspekte, wobei der Seitenschutz (21) aus mehreren Teilstrukturen (12.1, 12.2) zusammengesetzt ist, die lösbar, beispielsweise werkzeuglos lösbar, miteinander verbunden sind.
Aspekt 10 Schutzvorrichtung nach einem der vorhergehenden Aspekte in Kombination mit Aspekt 2, wobei die Absaugeinrichtung (12) aus mehreren Teilstrukturen (12.1, 12.2) besteht, die an einer oder mehreren Verbindungsstellen (12a, 12b) lösbar, beispielsweise werkzeuglos lösbar, miteinander verbunden sind.
Aspekt 11 Schutzvorrichtung nach vorhergehendem Aspekt, wobei die jeweilige Verbindungstelle (12a, 12b) eine Steck-, Klick- und/oder Bajonettverbindung ist.
Aspekt 12 Schutzvorrichtung nach einem der vorhergehenden Aspekte in Kombination mit Aspekt 3, wobei die Abdeckung (11) aus mehreren Teilstrukturen besteht, die lösbar, beispielsweise werkzeuglos lösbar, miteinander verbunden sind.
Aspekt 13 Schutzvorrichtung nach dem vorhergehenden Aspekt in Kombination mit den Aspekten 2 und 3, wobei die Absaugeinrichtung (12) und die Abdeckung (11) miteinander unbeweglich verbunden sind.
Aspekt 14 Schutzvorrichtung nach einem der vorhergehenden Aspekte in Kombination mit Aspekt 3, wobei die Abdeckung (11) relativ zum Seitenschutz (21) verstellbar ist.
Aspekt 15 Schutzvorrichtung nach einem der vorhergehenden Aspekte, wobei der Seitenschutz (21) oder nur ein oder mehrere Teilbereiche des Seitenschutzes (21) am Überkopfschutz (10) hängend angebracht ist oder sind.
Aspekt 16 Schutzvorrichtung nach einem der vorhergehenden Aspekte, wobei der Seitenschutz (21) oder nur ein oder mehrere Teilbereiche vorhangartig ausgebildet ist oder sind.
Aspekt 17 Schutzvorrichtung nach einem der vorhergehenden Aspekte, wobei der Seitenschutz (21) insgesamt oder nur ein oder mehrere Teilbereiche des Seitenschutzes (21) von Lamellen (22) gebildet wird oder werden.
Aspekt 18 Schutzvorrichtung nach einem der vorhergehenden Aspekte, wobei der Seitenschutz (21) insgesamt oder nur ein oder mehrere Teilbereiche des Seitenschutzes (21) von einer oder mehreren Folien, Maschenware oder einem der mehreren Netzen gebildet wird oder werden.
Aspekt 19 Schutzvorrichtung nach einem der vorhergehenden Aspekte, wobei die Lamellen nach Aspekt 17 oder die jeweilige Folie oder die Maschenware oder das jeweilige Netz nach Aspekt 18 transluzent oder transparent ist oder sind.
Aspekt 20 Schutzvorrichtung nach einem der Aspekte 1 bis 15, wobei der Überkopfschutz (10) und der Seitenschutz (21) einen zusammenhängenden Bereich einer Schutzhaube (25) bilden.
Aspekt 21 Schutzvorrichtung nach vorhergehendem Aspekt, wobei die Schutzhaube (25) einen oder mehrere Durchgriffe (26) für einen behandelnden Arzt aufweist.
Aspekt 22 Schutzvorrichtung nach einem der zwei unmittelbar vorhergehenden Aspekte, wobei an einer Innenwand im unteren Bereich der Schutzhaube (25) eine Auffangrinne (27) vorgesehen ist.
Aspekt 23 Schutzvorrichtung nach einem der vorhergehenden Aspekte, wobei die Schutzvorrichtung ein integraler oder nachträglich montierter Bestandteil des Behandlungsstuhls (1), vorzugsweise Zahnbehandlungsstuhls, ist.
Aspekt 24 Schutzvorrichtung nach einem der Aspekte 1 bis 22, wobei die Schutzvorrichtung unabhängig vom Behandlungsstuhl (1) abgestützt und relativ zur Patientenauflage (3) in der Behandlungsposition positionierbar ist.
Aspekt 25 Schutzvorrichtung nach einem der vorhergehenden Aspekte, wobei der Seitenschutz (21) sterilisierbar und/oder für den einmaligen Gebrauch austauschbar ausgebildet ist.
Aspekt 26 Schutzvorrichtung nach einem der vorhergehenden Aspekte, wobei der Überkopfschutz (10) den Behandlungsraum (20) nach oben begrenzt.
Aspekt 27 Schutzvorrichtung nach einem der vorhergehenden Aspekte, wobei der Behandlungsraum (20) zylinderförmig, insbesondere kreiszylinderförmig oder ellipsenzylinderförmig, ausgebildet ist.
Aspekt 28 Schutzvorrichtung nach einem der vorhergehenden Aspekte, wobei der Überkopfschutz (10) in wenigstens einem Gelenk (7, 9), beispielsweise Schubgelenk (7), relativ zum Gestell (6) oder dem Behandlungsstuhl (1) verstellbar ist.
Aspekt 29 Schutzvorrichtung nach einem der vorhergehenden Aspekte in Kombination mit Aspekt 2, wobei die Absaugeinrichtung (12) in wenigstens einem Gelenk (7, 9), beispielsweise Schubgelenk (7), relativ zum Gestell (6) oder dem Behandlungsstuhl (1) verstellbar, beispielsweise teleskopierbar, ist.
Aspekt 30 Schutzvorrichtung nach einem der vorhergehenden Aspekte in Kombination mit Aspekt 3, wobei die Abdeckung (11) in wenigstens einem Gelenk (7, 9), vorzugsweise Drehgelenk (9), relativ zum Gestell (6) und/oder relativ zur Absaugeinrichtung (12) nach Aspekt 2 verstellbar ist.
Aspekt 31 Schutzvorrichtung nach einem der vorhergehenden Aspekte, wobei der Überkopfschutz (10), beispielsweise die Abdeckung (11) nach Aspekt 3, zumindest bereichsweise aus Glas und/oder Plexiglas besteht.
Aspekt 32 Schutzvorrichtung nach einem der vorhergehenden Aspekte, wobei der Seitenschutz elastisch verformbar ist.
Aspekt 33 Schutzvorrichtung nach einem der vorhergehenden Ansprüche, wobei das Gestell (6) ein oder mehrere Gelenke (7, 9), beispielsweise ein oder mehrere Schubgelenke (7), für eine vertikale Verstellung der Schutzvorrichtung aufweist.
Aspekt 34 Schutzvorrichtung nach einem der vorhergehenden Aspekte, wobei das Gestell (6) mehrere Gestellabschnitte aufweist, die über ein oder mehrere Gelenke (7, 9) miteinander verbunden sind.
Aspekt 35 Schutzvorrichtung nach einem der vorhergehenden Aspekte, wobei die Schutzvorrichtung ein Leuchtelement (15) zur Ausleuchtung des Behandlungsraums (20), insbesondere zur Beleuchtung des Kopfes des Patienten, umfasst.
Aspekt 36 Schutzvorrichtung nach einem der vorhergehenden Aspekte, umfassend eine oder mehrere in den Behandlungsraum (20) gerichtete Kameras (16) zur optischen Erfassung der Behandlung.
Aspekt 37 Schutzvorrichtung nach dem vorhergehenden Aspekt, wobei die jeweilige Kamera (16) an der Absaugeinrichtung nach Aspekt 2 angeordnet, vorzugsweise verstellbar angeordnet ist.
Aspekt 38 Schutzvorrichtung nach einem der zwei unmittelbar vorhergehenden Aspekte, wobei die jeweilige Kamera mit einem Bildschirm zur Darstellung, vorzugsweise vergrößerten Darstellung, der Behandlung verbunden oder verbindbar ist, vorzugsweise drahtlos.
Aspekt 39 Schutzvorrichtung nach einem der vorhergehenden Aspekte, wobei die Schutzvorrichtung ein klangerzeugendes Schallelement (17), insbesondere einen Lautsprecher, umfasst.
Aspekt 40 Behandlungsstuhl, insbesondere Zahnarztstuhl, der eine Schutzvorrichtung nach einem der vorhergehenden Aspekte als integralen Bestandteil oder nachgerüsteten Zusatz umfasst.

Nachfolgend werden Ausführungsbeispiele der Erfindung anhand von Figuren erläutert. Am Ausführungsbeispiel offenbar werdende Merkmale bilden jeweils einzeln und in jeder Merkmalskombination die Gegenstände der Ansprüche und vorstehenden Aspekte weiter. Es zeigen:
- Figur 1: eine Seitenansicht eines ersten Ausführungsbeispiels einer Schutzvorrichtung;
- Figur 2: eine perspektivische Darstellung der Schutzvorrichtung des ersten Ausführungsbeispiels;
- Figur 3: eine Draufsicht auf die Schutzvorrichtung des ersten Ausführungsbeispiels;
- Figur 4: eine Schutzvorrichtung eines zweiten Ausführungsbeispiels in einer perspektivischen Sicht;
- Figur 5: eine Seitenansicht der Schutzvorrichtung des zweiten Ausführungsbeispiels;
- Figur 6: eine schematische Seitenansicht einer Schutzvorrichtung eines dritten Ausführungsbeispiels;
- Figur 7: eine schematische Seitenansicht einer Schutzvorrichtung eines vierten Ausführungsbeispiels;
- Figur 8: eine Draufsicht auf die Schutzvorrichtung des vierten Ausführungsbeispiels; und
- Figur 9: eine perspektivische Darstellung einer Schutzvorrichtung eines fünften Ausführungsbeispiels.

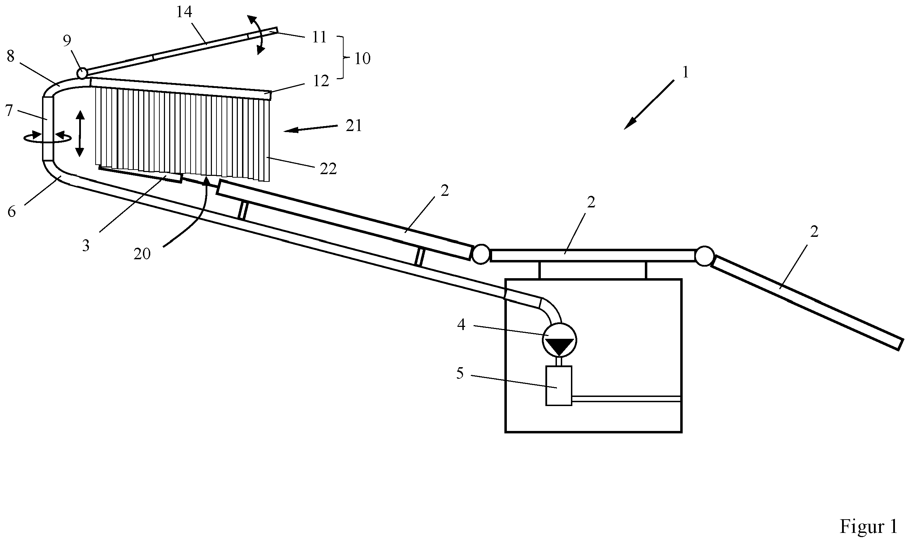

Figur 1 zeigt einen Behandlungsstuhl, beispielhaft einen Zahnarztstuhl, und eine Schutzvorrichtung eines ersten Ausführungsbeispiels. Der Behandlungsstuhl 1 umfasst Auflagen 2 für Beine und Rumpf eines Patienten und eine Kopfstütze 3. In einer Behandlungsposition liegt ein nicht dargestellter Patient so auf dem Behandlungsstuhl 1, dass die Beine und der Rumpf auf den Auflagen 2 und der Kopf auf der Kopfstütze 3 abgestützt sind.

Die Schutzvorrichtung umfasst einen Überkopfschutz 10 und einen nach unten, zur Kopfstütze 3 offenen Seitenschutz 21. Der Überkopfschutz 10 und der Seitenschutz 21 begrenzen gemeinsam einen Behandlungsraum 20. Innerhalb des Behandlungsraums 20 befindet sich der Kopf des nicht dargestellten Patienten. Die Schutzvorrichtung nimmt eine Behandlungsposition ein, in welcher der Kopf des Patienten in den Seitenschutz 21 ragt.

Der Überkopfschutz 10 und der Seitenschutz 21 können über ein Teleskopgelenk 7 eines Gestells 6 der Schutzvorrichtung in die und aus der Behandlungsposition bewegt werden. Vorteilhafterweise ist das Teleskopgelenk 7 derart ausgebildet, dass es unter externer Krafteinwirkung, beispielsweise durch Ziehen am und/oder durch Drücken auf den Überkopfschutz 10, eine vertikale Hubbewegung und optional eine horizontale Schwenkbewegung des Überkopfschutzes 10 und des Seitenschutzes 21 zulässt. Solange keine externe Kraft aufgebracht wird, hält das Gestell 6 die Schutzvorrichtung in der Behandlungsposition und/oder jeder anderen zuletzt gewählten Position.

Im ersten Ausführungsbeispiel ist die Schutzvorrichtung über das Gestell 6 mit dem Behandlungsstuhl 1 integral verbunden. Die Schutzvorrichtung des ersten Ausführungsbeispiels ist integraler Bestandteil des Behandlungsstuhls 1.

Der Überkopfschutz 10 umfasst eine Absaugeinrichtung 12 und eine Abdeckung 11. Die Abdeckung 11 ist über ein Gelenk 9 beweglich, im Beispiel klappbar, mit der Absaugeinrichtung 12 verbunden. In einem niedergeklappten Zustand deckt die Abdeckung 11 die Oberseite des Behandlungsraums 20 vollständig ab, so dass aus dem Behandlungsraum 20 keine Aerosole nach oben entweichen können. Die Abdeckung 11 ist zumindest teilweise durchsichtig ausgebildet. Sie kann, wie im Ausführungsbeispiel, insbesondere überall durchsichtig sein. Die Abdeckung 11 umfasst ein optisches Vergrößerungselement 14, beispielsweise eine Lupe 14. Im aufgeklappten Zustand kann die Abdeckung 11 an ihrer dem Behandlungsraum 20 zugewandte Unterseite gereinigt und/oder desinfiziert werden.

Die Absaugeinrichtung 12 ist an einem oberen Rand des Behandlungsraums 20, in einem Übergangsbereich zwischen dem Seitenschutz 21 und der niedergeklappten Abdeckung 11 angeordnet.

Die Absaugeinrichtung 12 ist ringförmig. Über die Absaugeinrichtung 12 wird aerosolhaltige Luft aus dem Behandlungsraum 20 abgesaugt. Aus der Umgebung strömt dabei Frischluft von unten und vorteilhafterweise auch seitlich durch den Seitenschutz 21 in den Behandlungsraum 20 nach. Die Absaugeinrichtung 12 ist über eine luftdichte Verrohrung des Gestells 6 oder eine schlauchförmige Verbindung, welche innerhalb einer Verrohrung des Gestells 6 und/oder außen am Gestell 6 verläuft, mit einer Pumpe 4 und einem Filter 5 verbunden.

Der Filter 5 ist dazu ausgebildet Viren, Keime, Bakterien und/oder andere Krankheitserreger aus der aus dem Behandlungsraum 20 abgesaugten, Luft heraus zu filtern, so dass die gefilterte Luft in die Umgebung abgegeben werden kann. Der Filter 5 und/oder die Pumpe 4 kann oder können integraler Bestandteil des Behandlungsstuhls 1, wie dargestellt, oder zusätzlich extern vorgesehen sein.

Der Seitenschutz 21 umfasst eine Vielzahl von Lamellen 22, welche jeweils vom Überkopfschutz 10 herabhängen. Die Lamellen 22 können an einer ringförmigen Haltervorrichtung des Überkopfschutzes 10, insbesondere an der Absaugeinrichtung 12, befestigt sein. Die Lamellen 22 weisen eine solche Länge auf, dass sie in der Behandlungsposition mindestens bis auf die Höhe der Kopfstütze 4 herab reichen und sich der Mundbereich des Patienten vollständig innerhalb des Behandlungsraums 20 befindet. Der von den Lamellen 22 gebildete Vorhang verhindert, dass aerosolhaltige Luft seitlich aus dem Behandlungsraum 20 entweichen kann. Gleichzeitig kann der behandelnde Arzt durch den Lamellenvorhang 22 in den Behandlungsraum 20 greifen. Aus hygienischen Gründen sind die Lamellen 22 sterilisierbar oder für den einmaligen Gebrauch ausgebildet.

Der Arzt kann den Mundraum des Patienten durch den von den Lamellen 22 bereitgestellten Seitenschutz 21 hindurch erreichen und gleichzeitig über die Abdeckung 11 in den Behandlungsraum 20, insbesondere in den Mundraum des Patienten, schauen. Aufgrund der Absaugeinrichtung 12, der Abdeckung 11 und des Seitenschutzes 21 kann sich die aerosolhaltige Luft nicht derart ausbreiten, dass der Behandelnde sie einatmet.

Anstelle der Lamellen 22 kann auch eine oder können gemeinsam mehrere Folien oder Maschenware oder andere netzartige Strukturen den Seitenschutz 21 in der Art eines Vorhangs bilden. Zweckmäßigerweise bilden in derartigen Ausführungen mehrere über den Umfang des Behandlungsraums 20 verteilt angeordnete Folienabschnitte oder Abschnitte aus Maschenware oder Abschnitte netzartiger Strukturen einen durchgreifbaren Seitenschutz, indem diese Strukturen (Folien, Maschenware, Netze) einander in einem oder mehreren Umfangsteilbereichen überlappen oder unmittelbar aneinander grenzen, so dass der behandelnde Arzt zwischen zwei benachbarten, vorzugsweise einander teilweise überlappenden Abschnitten, in den Behandlungsraum 20 greifen kann.

Die Lamellen 22 oder Folien oder Maschenware oder Netze sind in vorteilhaften Ausführungen transluzent, besonders bevorzugt transparent.

Die Abdeckung 11 ist in einem Gelenk 9 relativ zur Absaugeinrichtung 12 um eine Rotationsache R₁₁ zwischen einer ersten Position, dem aufgeklappten Zustand, und einer zweiten Position, dem nieder- oder zugeklappten Zustand, hin und her schwenkbar. Das Gelenk 9 kann, wie im Ausführungsbeispiel, zwischen einer Anschlussstruktur 8 des Gestells 6 und der Abdeckung 11 gebildet sein.

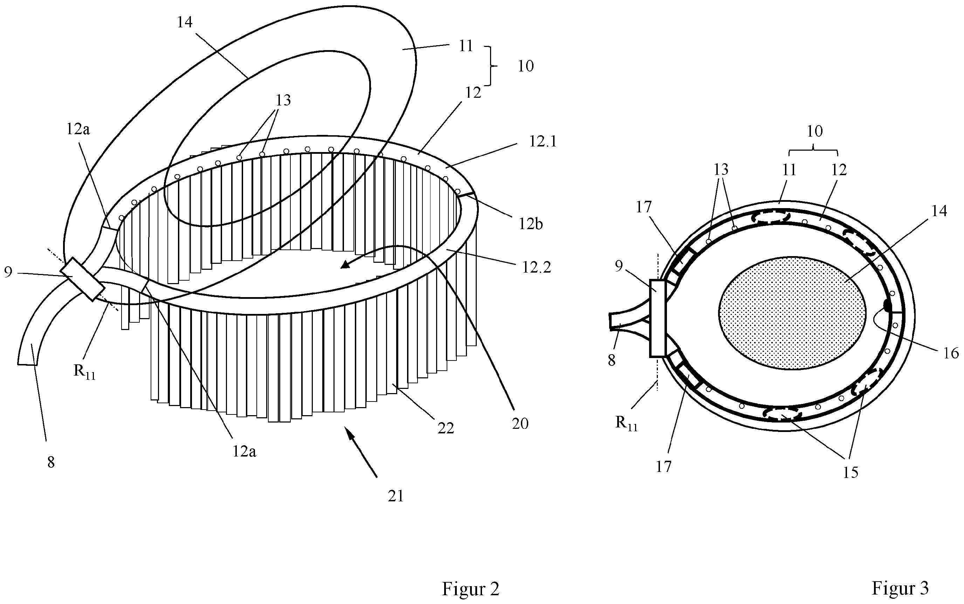

Figur 2 zeigt die Schutzvorrichtung des ersten Ausführungsbeispiels ohne Behandlungsstuhl in einer perspektivischen Sicht.

Die Absaugeinrichtung 12 umfasst eine Ringstruktur mit mehreren Absaugöffnungen 13, durch welche die Luft aus dem Behandlungsraum 20 in die Ringstruktur einströmen kann. Die Ringstruktur ist an Verbindungsstellen 12a mit der Anschlussstruktur 8 des Gestells 6 lösbar verbunden. Darüber hinaus kann die Ringstruktur in sich mehrteilig sein und mehrere voneinander lösbare Ringabschnitte aufweisen. Im Ausführungsbeispiel setzt sich die Ringstruktur aus zwei Ringabschnitten 12.1 und 12.2 zusammen. Die Ringabschnitte 12.1 und 12.2 sind an einer weiteren Verbindungsstelle 12b lösbar miteinander verbunden. Zum Reinigen und Desinfizieren, vorzugsweise Sterilisieren, werden die Ringabschnitte 12.1 und 12.2 an den Verbindungsstellen 12a und 12b voneinander und von der Anschlussstruktur 8 gelöst. Die jeweilige Verbindungsstelle 12a und 12b kann beispielsweise als Steck- oder Klick- oder Bajonettverbindung gebildet sein.

Die den Seitenschutz 21 bildenden Lamellen 22 hängen von der Absaugeinrichtung 12 herab, so dass der Seitenschutz 21 und der Überkopfschutz 10 einen ellipsenzylinderförmigen Behandlungsraum 20 begrenzen. Die Lamellen 22 können beispielsweise mittels Klett- und/oder Magnetverbindung mit der Absaugeinrichtung 12 verbunden sein.

Figur 3 zeigt die Schutzvorrichtung des ersten Ausführungsbeispiels in einer Draufsicht. Die Abdeckung 11 ist auf die Absaugeinrichtung 12 geklappt. Zu erkennen sind in den Figuren 1 und 2 nicht gezeigte Schallelemente 17, welche als Lautsprecher 17 gebildet sind. Über die Lautsprecher 17 kann der Patient beim Warten oder auch während der Behandlung mit beruhigender Musik beschallt werden. Die Lautsprecher 17 können an der Absaugeinrichtung 12 und/oder an einem zusätzlichen Halter des Überkopfschutzes 10 angeordnet sein.

Darüber hinaus umfasst die Schutzvorrichtung mehrere Leuchtelemente 15, die in den Figuren 1 und 2 zur Vereinfachung weggelassen wurden. Die Leuchtelemente 15 sind dazu ausgebildet, den Behandlungsraum 20 zu beleuchten. Vorteilhafterweise sind die Leuchtelemente 15 so ausgerichtet, dass sie in den Mundraum des Patienten ausleuchten können. Die Leuchtelemente 15 sind an der Absaugeinrichtung 12 und/oder an einem zusätzlichen Halter des Überkopfschutzes 10 angeordnet.

Die Schutzvorrichtung kann eine Kamera 16 oder auch mehrere Kameras umfassen. Die jeweilige Kamera 16 ist in den Behandlungsraum 20 gerichtet. Die jeweilige Kamera 16 kann an der Schutzvorrichtung, beispielsweise wie im Ausführungsbeispiel an der Ringstruktur der Absaugeinrichtung 12, verstellbar angeordnet sein, um die Ausrichtung relativ zum Patienten bzw. relativ zur Kopfstütze 3 (Figur 1) verstellen zu können. Die eine oder die mehreren Kameras 16 kann oder können mit einem Bildschirm verbunden sein, um die Behandlung live, in Echtzeit, auf dem Bildschirm darstellen und vorzugsweise vergrößert darstellen zu können. Die Darstellung am Bildschirm kann in derartigen Ausführungen bei der Behandlung unterstützend eingesetzt werden. Alternativ oder zusätzlich kann oder können die Aufnahmen der einen oder mehreren Kameras 16 gespeichert und zu Dokumentationszwecken oder Lehrzwecken oder Forschungszwecken gespeichert und zu einem späteren Zeitpunkt dargestellt werden.

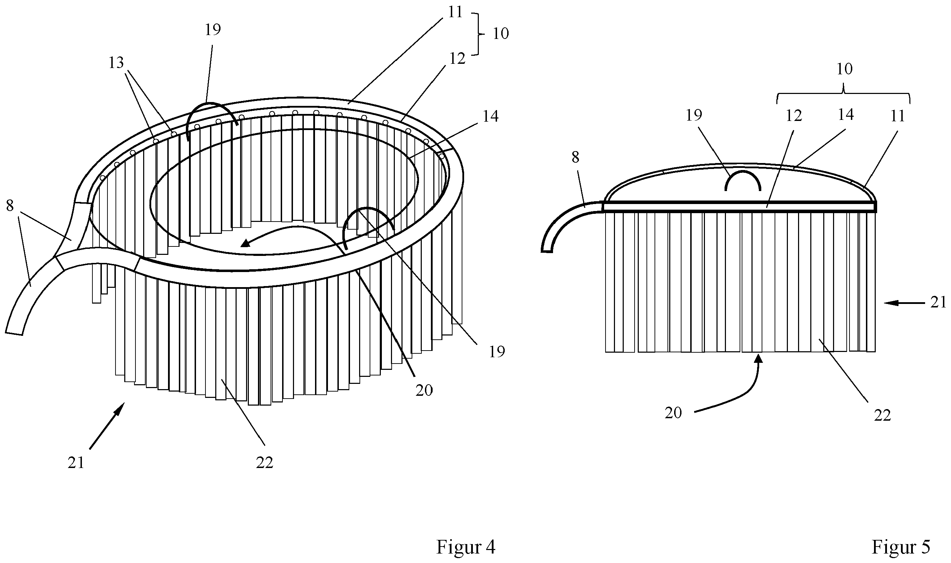

Die Figuren 4 und 5 zeigen eine Schutzvorrichtung eines zweiten Ausführungsbeispiels in einer perspektivischen Sicht und einer Seitenansicht. Das dritte Ausführungsbeispiel unterscheidet sich in der Form der Abdeckung 11 vom ersten Ausführungsbeispiel. Wie insbesondere in Figur 5 erkennbar, ist die Abdeckung 11 des zweiten Ausführungsbeispiels haubenförmig und nicht plattenförmig bzw. flach ausgebildet. Aufgrund der haubenförmigen Abdeckung 11 ist das Volumen des Behandlungsraums 20 im Vergleich zum ersten Ausführungsbeispiel vergrößert, was für den Patienten angenehm ist.

Ferner umfasst die Abdeckung 11 Griffstücke 19, welche von der Oberseite der Abdeckung 11, nämlich der dem Behandlungsraum 20 abgewandten Seite der Abdeckung 11, vorragen. Über die Griffstücke 19 kann der Arzt die Schutzvorrichtung in die Behandlungsposition und aus der Behandlungsposition bewegen. Die Abdeckung 11 kann auf der Absaugeinrichtung 12 lose aufliegen oder in einer leicht lösbaren Rastverbindung oder dergleichen arretiert sein.

Das Gelenk 9 (Figuren 1 bis 3) ist im zweiten Ausführungsbeispiel entfallen.

Im Übrigen entspricht die Schutzvorrichtung des zweiten Ausführungsbeispiels dem ersten Ausführungsbeispiel. In einer Modifikation kann die Abdeckung 11 auch im zweiten Ausführungsbeispiel über ein Gelenk, vergleichbar dem Gelenk 9 des ersten Ausführungsbeispiels, der Anschlussstruktur 8 oder direkt mit der Ringstruktur der Absaugeinrichtung 12 klappbar verbunden sein. In diesem Fall können die Griffstücke 19 zusätzlich oder alternativ dazu verwendet werden, die Abdeckung 11 zwischen einem aufgeklappten Zustand und einem zugeklappten Zustand hin und her zu bewegen.

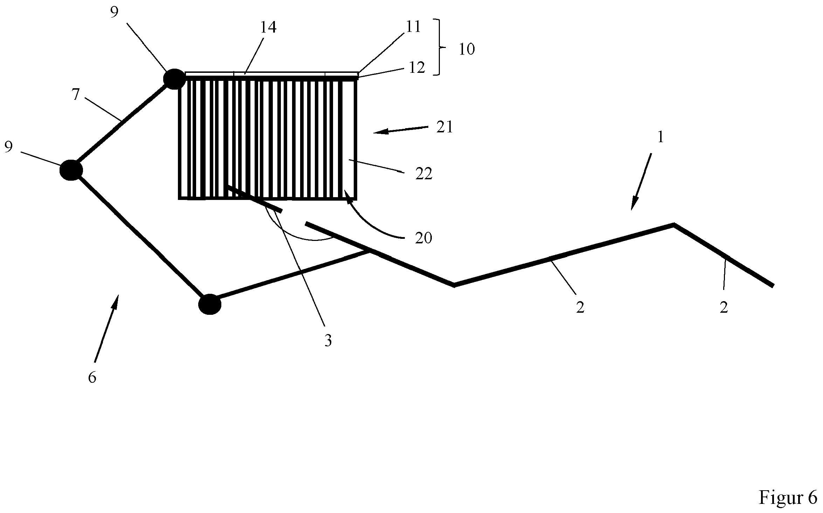

Figur 6 ist eine schematische Darstellung einer Schutzvorrichtung eines dritten Ausführungsbeispiels, in welchem die Schutzvorrichtung als nachgerüsteter Zusatz des Behandlungsstuhls 1 gebildet ist. Wie dargestellt ist das Gestell 6 der Schutzvorrichtung über mehrere Streben und Gelenke 9 und/oder einem Teleskopgelenk 7 mit dem Behandlungsstuhl 1 verbunden. Insbesondere die Gelenke 6 und das Teleskopgelenk 7 ermöglichen es, dass die Schutzvorrichtung in die und aus der Behandlungsposition bewegt werden kann und während der Behandlung in der Behandlungsposition gehalten wird.

Im dritten Ausführungsbeispiel umfasst die Schutzvorrichtung einen Überkopfschutz 10 und einen Seitenschutz 21 entsprechend dem ersten Ausführungsbeispiel.

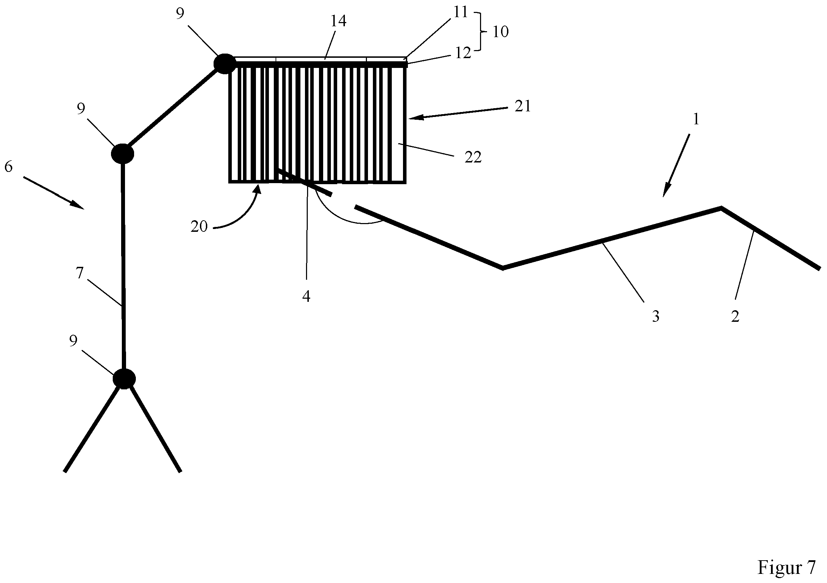

Figur 7 ist eine schematische Darstellung einer Schutzvorrichtung eines vierten Ausführungsbeispiels, in welchem die Schutzvorrichtung als individuelle, mithin vom Behandlungsstuhl 1 unabhängige, Vorrichtung gebildet ist. Die Schutzvorrichtung umfasst ein Gestell 6, welches unabhängig vom Behandlungsstuhl 1 im Raum positioniert werden kann. Dies ermöglicht, dass die Schutzvorrichtung auch in Kombination mit Behandlungsstühlen 1 verwendet werden kann, bei welchen eine Verbindung des Gestells 6 mit dem Behandlungsstuhl 1 technisch nicht möglich bzw. zu aufwändig ist. Auch im vierten Ausführungsbeispiel ist die Schutzvorrichtung über Streben, Gelenke 9 und/oder ein Teleskopgelenk 7 in die Behandlungsposition bewegbar und wird in der Behandlungsposition gehalten.

Im vierten Ausführungsbeispiel umfasst die Schutzvorrichtung einen Überkopfschutz 10 und einen Seitenschutz 21, die dem ersten Ausführungsbeispiel entsprechen.

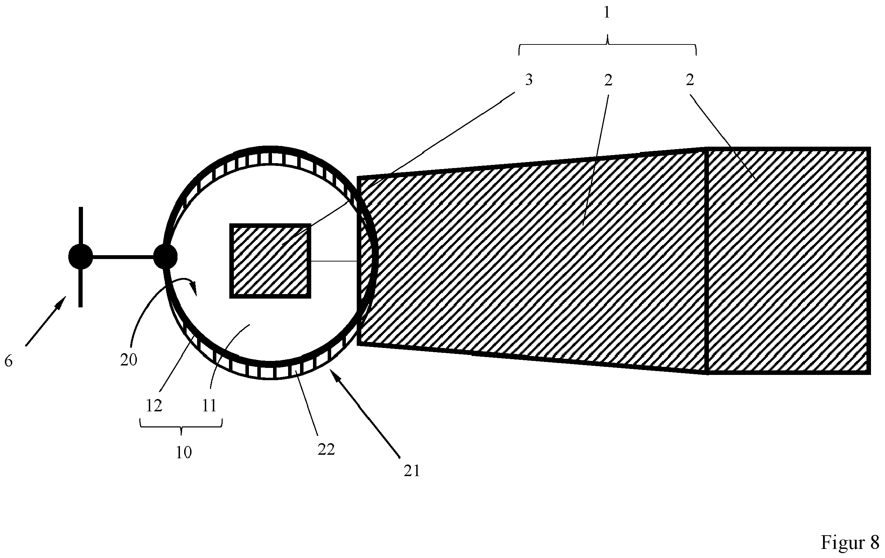

Figur 8 zeigt eine Draufsicht auf das vierte Ausführungsbeispiel der Schutzvorrichtung. Die Abdeckung 11 befindet sich im zugeklappten Zustand und weist keine Lupe auf. Der Arzt, welcher von oben durch die Abdeckung 11 schaut, kann ein optisches Vergrößerungselement anderweitig bereitstellen.

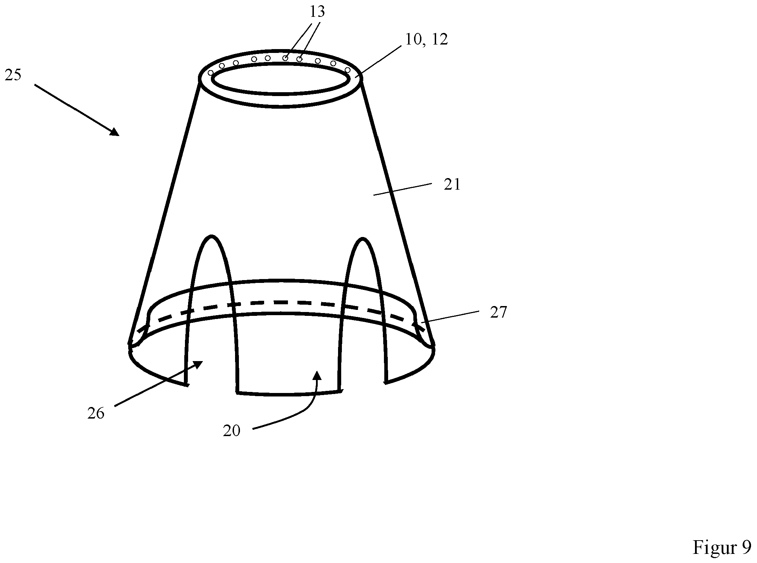

Figur 9 zeigt eine Schutzvorrichtung eines fünften Ausführungsbeispiels in einer perspektivischen Sicht. Im fünften Ausführungsbeispiel bilden ein Überkopfschutz 10 und ein Seitenschutz 21 einen zusammenhängenden Bereich einer Schutzhaube 25.

Der Seitenschutz 21 verjüngt sich von seiner offenen Unterseite nach oben, beispielhaft konisch. Der Seitenschutz 21 ist zumindest zu einem Großteil durchsichtig. Darüber hinaus weist der Seitenschutz 21 Durchgriffe 26 auf, durch welche der Arzt während der Behandlung greifen kann, um mit den Händen in den durch den Seitenschutz 21 und den Überkopfschutz 10 begrenzten Behandlungsraum 20 zu gelangen.

An oder nahe der Unterseite ist am Innenumfang des Seitenschutzes 21 eine Auffangrinne 27 angeordnet. Die Auffangrinne 27 ist dazu ausgebildet Flüssigkeit, welche sich an der Innfläche des Seitenschutzes 21 sammeln und an dieser aufgrund der Schwerkraft nach unten fließen kann, aufzufangen und optional an einer bestimmten Stelle aus der Schutzvorrichtung abzuleiten.

Der Überkopfschutz 10 befindet sich am oberen Ende des Seitenschutzes 21 und umfasst eine ringförmige Absaugeinrichtung 12 mit Absaugöffnungen 13. Der Seitenschutz 21 ist im Vergleich zu den anderen Ausführungsbeispielen nach oben verlängert. Aufgrund des sich verjüngenden Seitenschutzes 21 weist die Absaugeinrichtung 12 einen verhältnismäßig geringen Durchmesser auf. Dies begünstigt die Absaugung. Auf eine zusätzliche Abdeckung 11 kann leichter als in den anderen Ausführungsbeispielen verzichtet werden.

### Bezugszeichen

- 1: Behandlungsstuhl
- 2: Auflage, Beine und Rumpf
- 3: Kopfstütze
- 4: Pumpe
- 5: Filter
- 6: Gestell
- 7: Gelenk, translatorisch, Teleskopgelenk
- 8: Anschlussstruktur
- 9: Gelenk, rotatorisch, Scharnier
- 10: Überkopfschutz
- 11: Abdeckung
- 12: Absaugeinrichtung
- 12.1: Ringabschnitt
- 12.2: Ringabschnitt
- 12a: Verbindungsstelle
- 12b: Verbindungsstelle
- 13: Absaugöffnung
- 14: Optisches Vergrößerungselement
- 15: Leuchtelement
- 16: Kamera
- 17: Schallelement
- 18: -
- 19: Griffstück
- 20: Behandlungsraum
- 21: Seitenschutz
- 22: Lamelle
- 23: -
- 24: -
- 25: Schutzhaube
- 26: Durchgriffe
- 27: Auffangrinne

- R₁₁: Rotationsachse

## Patentansprüche

1. Schutzvorrichtung zum Schutz vor Aerosolausbreitung bei medizinischen Behandlungen am Kopf, insbesondere bei zahnmedizinischen Behandlungen, die Schutzvorrichtung umfassend:
(a) einen Überkopfschutz (10) zur Verhinderung einer Ausbreitung von Aerosolen nach oben,
(b) einen nach unten offenen Seitenschutz (21), der einen Behandlungsraum (20) an einem Seitenumfang begrenzt, um eine seitliche Ausbreitung der Aerosole zu verhindern, und
(c) ein Gestell (6) zum Positionieren des Überkopfschutzes (10) und des Seitenschutzes (21) relativ zu einer Patientenauflage (3) eines Behandlungsstuhls (1) in einer Behandlungsposition und Halten des Überkopfschutzes (10) und des Seitenschutzes (21) in der Behandlungsposition,
(d) wobei der Seitenschutz (21) von außen nach innen in den Behandlungsraum (20) durchgreifbar ist.

2. Schutzvorrichtung nach dem vorhergehenden Anspruch, wobei der Überkopfschutz (10) eine Absaugeinrichtung (12) zum Absaugen von Luft und Aerosolen aus dem Behandlungsraum (20) umfasst.

3. Schutzvorrichtung nach einem der vorhergehenden Ansprüche, wobei der Überkopfschutz (10) eine zumindest bereichsweise transparente Abdeckung (11), die den Seitenschutz (21) nach oben zumindest teilweise abdeckt, umfasst.

4. Schutzvorrichtung nach einem der vorhergehenden Ansprüche in Kombination mit Anspruch 2, wobei die Absaugeinrichtung (12) eine fluidleitende Absaugstruktur, vorzugsweise Ringstruktur, mit einer oder mehreren Absaugöffnungen (13) umfasst.

5. Schutzvorrichtung nach einem der vorhergehenden Ansprüche in Kombination mit Anspruch 2, wobei die Absaugeinrichtung (12) an eine bezüglich der Schutzvorrichtung externe Absaugeinrichtung (4, 5), beispielsweise an eine Absaugeinrichtung (4, 5) des Behandlungsstuhls (1), angeschlossen oder anschließbar ist.

6. Schutzvorrichtung nach einem der vorhergehenden Ansprüche in Kombination mit Anspruch 3, wobei die Abdeckung (11) ein optisches Vergrößerungselement (14), beispielsweise eine Lupe (14), aufweist.

7. Schutzvorrichtung nach einem der vorhergehenden Ansprüche in Kombination mit den Ansprüchen 2 und 3, wobei die Absaugeinrichtung (12) und/oder die Abdeckung (11) in wenigstens einem Gelenk (7, 8) relativ zum Gestell (6) und/oder relativ zur Patientenauflage (3) verstellbar ist/sind.

8. Schutzvorrichtung nach einem der vorhergehenden Ansprüche, wobei der Seitenschutz (21) am Überkopfschutz (10) hängend angebracht ist.

9. Schutzvorrichtung nach einem der vorhergehenden Ansprüche, wobei der Seitenschutz (21) oder nur ein oder mehrere Teilbereiche des Seitenschutzes (21) vorhangartig ausgebildet ist oder sind, wobei der Seitenschutz (21) oder nur der eine oder die mehreren vorhangartigen Teilbereiche beispielsweise von Lamellen (22) oder einer oder mehreren Folien oder einem oder mehreren Netzen gebildet wird oder werden, wobei die jeweilige Lamelle oder Folie oder das jeweilige Netz vorzugsweise transluzent oder transparent ist.

10. Schutzvorrichtung nach einem der Ansprüche 1 bis 8, wobei der Überkopfschutz (10) und der Seitenschutz (21) einen zusammenhängenden Bereich einer Schutzhaube (25) bilden, die vorzugsweise einen oder mehrere Durchgriffe (26) für einen behandelnden Arzt aufweist.

11. Schutzvorrichtung nach einem der vorhergehenden Ansprüche, wobei zumindest ein Teil des Seitenschutzes (21) und/oder ein Teil (11, 12) des Überkopfschutzes (10) zerstörungsfrei und vorzugsweise ohne Werkzeug vom Gestell (6) lösbar ist.

12. Schutzvorrichtung nach einem der vorhergehenden Ansprüche, wobei der Seitenschutz (21) und/oder der Überkopfschutz (10), vorzugsweise die Absaugeinrichtung (12) nach Anspruch 2 und/oder die Abdeckung (11) nach Anspruch 3, aus mehreren Teilstrukturen (12.1, 12.2) besteht oder bestehen und die Teilstrukturen (12.1, 12.2) lösbar, beispielsweise werkzeuglos lösbar, miteinander verbunden sind.

13. Schutzvorrichtung nach einem der vorhergehenden Ansprüche, umfassend:
- ein Leuchtelement (15) zum Ausleuchten des Behandlungsraums (20) oder wenigstens eines Teilbereichs des Behandlungsraums (20), und/oder
- eine in den Behandlungsraum (20) gerichtete Kamera (16) zum optischen Erfassen der Behandlung, und/oder
- einen Lautsprecher (17) zum Beschallen des Behandlungsraums (20), vorzugsweise mit Musik.

14. Schutzvorrichtung nach einem der vorhergehenden Ansprüche, wobei die Schutzvorrichtung integraler Bestandteil des Behandlungsstuhls (1) oder nachträglich am Behandlungsstuhl (1) montiert oder montierbar ist oder unabhängig vom Behandlungsstuhl (1) abgestützt und relativ zur Patientenauflage (3) in der Behandlungsposition positionierbar ist.

15. Behandlungsstuhl (1), insbesondere Zahnbehandlungsstuhl (1), der eine Schutzvorrichtung nach einem der vorhergehenden Ansprüche als integralen Bestandteil oder nachgerüsteten Zusatz umfasst.
